# EUROPÄISCHE PATENTSCHRIFT

(11) **EP 0 601 381 B1**
(45) Veröffentlichungstag und Bekanntmachung des Hinweises auf die Patenterteilung: **27.08.1997**
(21) Anmeldenummer: 93118813.0
(22) Anmeldetag: 23.11.1993
(51) Int. Cl.: C07D 239/30

(54) **Verfahren zur Herstellung von 2,5-Dibrompyrimidin**
Process for the production of 2,5 dibromopyridine
Procédé pour la préparation de 2,5 dibromopyridine

(30) Priorität: 24.11.1992 DE 4239413
(43) Veröffentlichungstag der Anmeldung: 15.06.1994
(73) Patentinhaber: HOECHST AKTIENGESELLSCHAFT, 65929 Frankfurt am Main (DE)
(72) Erfinder: Schlosser, Hubert, Dr., D-61479 Glashütten/Taunus (DE); Wingen, Rainer, Dr., D-65795 Hattersheim/Main (DE)

(56) Entgegenhaltungen:
- The chemistry of heterocyclic compounds, volume 16, D.J. BROWN, 1970, page 10, "The Pyrimidines Supplement I"
- The chemistry of heterocyclic compounds, volume 16, D.J. BROWN, 1985, page 11, "The Pyrimidines Supplement II"

## Beschreibung

2,5-Dibrompyrimidin ist ein wichtiges Vorprodukt, das z.B. zur Herstellung von Pflanzenschutzmitteln (z.B. WO 92/08714) oder Flüssigkristallen (z.B. WO 92/12974; DE-Anmelde-Nrn. von P 42 36 104.4, P 42 36 103.6, P 42 36 106.0) Verwendung finden kann.

Seine Herstellung ist beschrieben, z.B. in J.Chem.Soc. 1971, 1889, durch Umsetzung von 5-Brom-2-hydroxy-pyrimidin [s. J.Org.Chem. 25, 1916 (1960)] mit einem weit überstöchiometrischem Gemisch aus Phosporylbromid und Phosphortribromid.
Die Isolierung des Reaktionsproduktes erfolgt durch Hydrolyse, was unter ökologischen Aspekten, aber natürlich auch unter ökonomischen Gesichtspunkten, vor allem angesichts des großen Überschusses teurer Phosphorbromide, stark verbesserungswürdig ist.
Es besteht daher ein Bedarf nach einer Synthese für 2,5-Dibrompyrimidin mit preiswerten und gegebenenfalls wiederverwertbaren Reagenzien.

2-Chlor-pyrimidine lassen sich aus den entsprechenden 2-Hydroxy-pyrimidinen durch Umsetzung mit dem preiswerten Phosphoroxychlorid herstellen. Stand der Technik zur Überführung von 2-Chlor-pyrimidinen in 2-Brom-pyrimidine ist die Umsetzung mit dem teuren Phosphortribromid (J.Chem.Soc. C 1967, 1204). Somit besteht weiterhin ein Bedarf nach preiswerten Reagenzien zur Herstellung von 2-Brom-pyrimidin-Verbindungen.

Überraschenderweise wurde nun gefunden, daß sich 5-Brom-2-chlor-pyrimidin preiswert und mit sehr guten Ausbeuten in 2,5-Dibrom-pyrimidin überführen läßt mittels einer Lösung von Bromwasserstoff in nicht-wäßrigen Säuren.

Man arbeitet vorzugsweise bei einer Temperatur zwischen 0 und 120°C, besonders bevorzugt zwischen 20 und 70°C und ganz besonders bevorzugt zwischen 30 und 50°C. Bevorzugt erfolgt die Reaktion in Carbonsäuren, besonders bevorzugt in Alkansäuren mit vorzugsweise 1 bis 5 C-Atomen, wobei der Säurerest auch Fluoratome enthalten kann, und ganz besonders bevorzugt in Ameisensäure, Essigsäure, Propionsäure oder Trifluoressigsäure.
Die Isolierung des Reaktionsproduktes aus der Reaktionsmischung kann erfolgen durch Fällung mit einem Lösungsmittel, bevorzugt Wasser, oder durch destillative Entfernung der nicht-wäßrigen Säure.
Das erhaltene Rohprodukt kann gereinigt werden durch Standardmethoden, z.B. Umfällung, Destillation, Umkristallisation, Sublimation oder chromatographische Methoden.

Die Erfindung wird verdeutlicht durch die nachfolgenden Beispiele:

### Beispiel 1

Eine Suspension von 67,5 g 5-Brom-2-chlor-pyrimidin in 450 ml Bromwasserstoff (33 Gew.-%) in Eisessig wird 1 h auf 30°C und anschließend 0,5 h auf Siedetemperatur erhitzt. Im Vakuum wird eingeengt, die erhaltene Suspension auf die 5fache Menge Wasser gegeben, der erhaltene Feststoff abfiltriert und getrocknet.
Man erhält 51,4 g 2,5-Dibrom-pyrimidin entsprechend einer Ausbeute von 62 % der Theorie in einer Reinheit (HPLC) > 95 %.
Nach Schmelzpunkt (81 - 83°C), IR-Spektrum und ¹H-NMR-Spektrum ist das Produkt identisch mit dem nach J.Org.Chem. 25, 1916 (1960) hergestellten 2,5-Dibrom-pyrimidin.

### Beispiel 2

Eine Suspension von 135 g 5-Brom-2-chlor-pyrimidin in 1250 ml einer 33 gew.-%igen Lösung von Bromwasserstoff in Eisessig wird 6 h bei 20°C gerührt. Nach dieser Zeit wird in die 10fache Menge Wasser gegossen, der ausgefallene Feststoff abgetrennt und getrocknet.
Man erhält 117 g 2,5-Dibrom-pyrimidin, das den Spezifikationen von Beispiel 1 entspricht.

## Patentansprüche

1. Verfahren zur Herstellung von 2,5-Dibrom-pyrimidin, dadurch gekennzeichnet, daß 5-Brom-2-chlor-pyrimidin mit einer Lösung von Bromwasserstoff in einer nicht-wäßrigen Säure bei einer Temperatur zwischen 0 und 120°C umgesetzt wird.

2. Verfahren nach Anspruch 1, dadurch gekennzeichnet, daß die nichtwäßrige Säure eine Carbonsäure ist.

3. Verfahren nach Anspruch 1, dadurch gekennzeichnet, daß die Carbonsäure eine Alkansäure ist.

4. Verfahren nach Anspruch 1, dadurch gekennzeichnet, daß die Alkansäure Ameisensäure, Essigsäure, Propionsäure oder Trifluoressigsäure ist.

5. Verfahren nach den Ansprüchen 1 - 4, dadurch gekennzeichnet, daß die Reaktionstemperatur zwischen 20 und 70°C liegt.

6. Verfahren nach den Ansprüchen 1, 2 oder 3, dadurch gekennzeichnet, daß die Reaktionstemperatur zwischen 30 und 50°C liegt.

## Claims

1. A process for preparing 2,5-dibromopyrimidine, which comprises reacting 5-bromo-2-chloropyrimidine with a solution of hydrogen bromide in a non-aqueous acid at a temperature between 0 and 120°C.

2. The process as claimed in claim 1, wherein the non-aqueous acid is a carboxylic acid.

3. The process as claimed in claim 1, wherein the carboxylic acid is an aikanoic acid.

4. The process as claimed in claim 1, wherein the alkanoic acid is formic acid, acetic acid, propionic acid or trifluoroacetic acid.

5. The process as claimed in claims 1-4, wherein the reaction temperature is between 20 and 70°C.

6. The process as claimed in claims 1, 2 or 3, wherein the reaction temperature is between 30 and 50°C.

## Revendications

1. Procédé pour préparer la 2,5-dibromopyrimidine, caractérisé en ce qu'on fait réagir de la 5-bromo-2-chloropyrimidine avec une solution de bromure d'hydrogène dans un acide non-aqueux à une température de 0 à 120°C.

2. Procédé selon la revendication 1, caractérisé en ce que l'acide non-aqueux est un acide carboxylique.

3. Procédé selon la revendication 1, caractérisé en ce que l'acide carboxylique est un acide alcanoïque.

4. Procédé selon la revendication 1, caractérisé en ce que l'acide alcanoïque est l'acide formique, l'acide acétique, l'acide propionique ou l'acide trifluoracétique.

5. Procédé selon les revendications 1 à 4, caractérisé en ce que la température de réaction est de 20 à 70°C.

6. Procédé selon les revendications 1, 2 ou 3, caractérisé en ce que la température de réaction est comprise entre 30 et 50°C.
